# EUROPEAN PATENT SPECIFICATION

(11) **EP 0 943 617 B1**
(45) Date of publication and mention of the grant of the patent: **06.08.2003**
(21) Application number: 99301361.4
(22) Date of filing: 24.02.1999
(51) Int. Cl.: C07D 489/02

(54) **Process for the preparation of opiates**
Verfahren zur Herstellung von Opiaten
Procédé pour la préparation d'opiates

(30) Priority: 17.03.1998 GB 9805516
(43) Date of publication of application: 22.09.1999
(73) Proprietor: JOHNSON MATTHEY PUBLIC LIMITED COMPANY, London SW1Y 5BQ (GB)
(72) Inventor: Sebastian, Alice, Deptfort, NJ 08096 (US)
(74) Representative: Wishart, Ian Carmichael

(56) References cited:
- DE-C- 408 870
- US-A- 2 654 756
- US-A- 4 277 604
- DATABASE XFIRE Beilstein REaction ID 834398, XP002106974 & KNORR: CHEM.BER., vol. 36, 1903, page 3070
- I SEKI: "Studies on the Morphine Alkaloids and Its Related Compounds. XVII One-Step Preparations of Enol Ether and Pyrrolidinyl Dienamine of Normorphinone Derivatives" CHEMICAL AND PHARMACEUTICAL BULLETIN, vol. 18, no. 4, 1 January 1970, pages 671-676, XP002078242
- FATIADI SYNTHESIS February 1976, pages 66 - 67

## Description

The present invention relates to a novel process for the preparation of codeinone and analogues thereof.

Codeinone is a key intermediate for the synthesis of many morphinoid compounds. It is therefore desirable for a simple, straightforward and cost effective process for its preparation.

In earlier works, codeinone was obtained from thebaine by treating it with dry HBr in CH₂Cl₂ at -25°, followed by hydrolysis with water and dehydrobrominating with cold NaOH (Vesely, Z., Hodkova, J. and Trojanek J., Cesk. Farm. 1986, 35, 222-6). In another method, thebaine was converted to a mixture of codeinone and neopinone in aqueous HCO₂H containing Hg(OAc)₂ (US 4,277,604). However, thebaine is very expensive and does not occur naturally in a high yield; therefore the preparation of codeinone from thebaine is not an attractive method.

An alternative process for the preparation of codeinone is the direct oxidation of codeine using variety of reagents, such as silver carbonate (Rapoport, H. and Reist, N.H., J. Am. Chem. Soc. 1955, 77, 490-491) and Jones' reagent (Findlay, J.W.A., Butz, R.F. and Jones, E.C., Clin. Chem. 1981, 27, 1524-1535); however, these reagents only gave codeinone in poor yield. Only Oppenauer oxidation (US 2,654,756) has given codeinone in reasonably good yield and high purity. All these methods have their drawbacks, however. Oxidation with silver carbonate is not preferred due to its cost and incomplete reaction, whereas in the case of Jones' oxidation and Oppenauer oxidation the isolation of the product is difficult and complex.

The direct oxidation of codeine with active manganese dioxide (Ninan, A. and Sainsbury, M., Tetrahedron, 1992, 48, 6709) and γ-manganese dioxide (Highet, R.J. and Wildman, W.C., J. Am. Chem. Soc., 1955, 77, 4399) has also been reported. However, in both cases pure codeinone was obtained in only low yield due to the formation of 14β-hydroxycodeinone and low conversion.

The direct oxidation of codeine with chromate reagents in acidic conditions has also been reported (Knorr, Chem. Ber. 1903, 36, 3070 and DE 408870). Codeinone was obtained in low or very low yields.

The object of the present invention is to provide a simple process for the preparation of codeinone and analogues thereof, and which provides the desired product in high yield and purity. The present inventors have found that if oxidation of codeine or a salt thereof, for example the phosphate salt, is carried out at a pH greater than 0.1 and less than 4.5, and the oxidation reagent is manganese dioxide, a high yield of codeinone is obtained which is not obtainable by any of the methods heretofore disclosed.

Accordingly, the present invention provides a novel process for the preparation of a compound of formula (I) 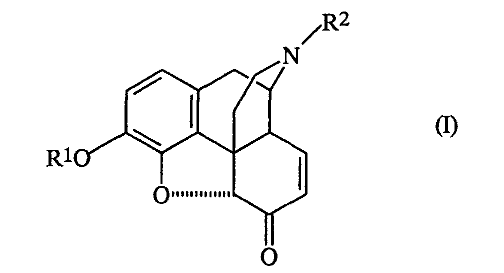 wherein R¹ is C₁₋₆ alkyl or a group 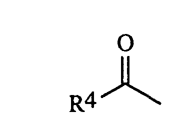 wherein R⁴ is C₁₋₆ alkyl, C₁₋₆ alkyl substituted by halogen or phenyl, or phenyl; and
R² is C₁₋₆ alkyl, allyl or C₁₋₆ alkyl substituted by cycloalkyl;
comprising the oxidation of a compound of formula (II), 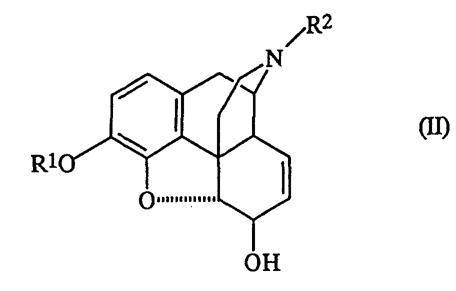 or a salt thereof, wherein R¹ and R² are as hereinbefore defined; and wherein and the oxidation reagent is manganese dioxide, characterised in that the oxidation is carried out in an acidic environment, at a pH or greater than 0.1 and less than 4.5.

Preferably, each of R¹ and R² may be the same or different and each is C₁₋₆ alkyl, such as C₁₋₄ alkyl, for example methyl or ethyl and preferably ethyl.

When the compound of formula (II) is present as a salt, preferably the salt is the phosphate salt (H₃PO₄).

The oxidation is carried out at a pH of from 0.1 < pH > 4.5; most preferably the oxidation is carried out at a pH of from 0.8 to 1.2.

The solvent may be any solvent suitable for use in such an oxidation reaction. Acetone was found to be the most suitable solvent for giving the desired product in high yield and high purity. Other solvents were also found to be particularly useful and were preferred in the order: acetone > THF > IPA > CH₃CN > NMP. Preferably, the solvent is used as a 50:50 (v/v) solvent/water mixture.

The oxidation reagent is manganese dioxide, such as activated manganese dioxide or γ-manganese dioxide, preferably γ-manganese dioxide. Suitably, the oxidation reagent is present in an amount of at least 3 equivalents or more with respect to the compound of formula (I).

The invention will now be further described by way of example only.

### General Procedure for the Preparation of Codeinone from Codeine Phosphate or Codeine

A solution of codeine or codeine phosphate was dissolved in the appropriate solvent system and a specified volume of hydrochloric acid of varying concentration was added. Manganese dioxide (either freshly prepared γ-manganese dioxide or activated manganese dioxide from Aldrich) was added and the reaction mixture was stirred at ambient temperature for 1.5 to 4 hours. The progress of the reaction was monitored by HPLC. The reaction mixture was filtered through a celite pad, washed with additional solvent or water, and neutralised with ammonium hydroxide. The product was extracted with methylene chloride (3 x 150ml) and the combined extracts washed with water and dried over anhydrous sodium sulphate. The organic layer was then evaporated to yield codeinone.

In all the experiments the reaction was followed by HPLC and the retention time of the product was compared with that of the standard. In some cases the crude product was quantitated to obtain the purity. The HPLC analysis was done on a Shimadzu system. The column used was Phenomenex's Prodigy 5µ ODS (3) 100°A, 250 x 4.6mm. The method was isocratic. The mobile phase was: water (1450ml) + acetonitrile (550ml) + Et₃N (2ml) + 1.73g of 1-octanesulphonate, sodium salt + pH 3.5 (adjusted with 85% phosphoric acid). In the cases where the product was isolated, it was also characterised by comparing its ¹H NMR with that of the standard: ¹H NMR (CDCl₃) δ 1.85-1.92(1H, m, 15_{α}), 2.05-2.15(1H, m, 16_{α}), 2.27-2.4(2H, m, 15_{β}, 10_{α}), 2.45(3H, s, N-CH₃), 2.58-2.62(1H, m, H_{16β}), 3.07-3.14(1H, d, H_{10β}), 3.19-3.21(1H, d, H_{9α}), 3.39-3.44(1H, m, H₁₄), 3.84(3H, s, OCH₃), 4.70(1H, s, H_{5β}), 6.05-6.11(1H, dd, H₈), 659-6.70(3H, m, H₁, H₂, H₇).

The following examples were carried out using the starting material and reagents indicated.

| | Starting Material (g) | Solvent System (ml) | Acid (ml) | Oxidant (g) | pH | Yield (%) | Purity (HPLC area %) |
|---|---|---|---|---|---|---|---|
| Example 1 | Codeine | IPA (14) | 6N HCl | γ-MnO₂ | 1.62 | 94 | ∼95 |
| | (05) | H₂O(14) | (0.5) | (1.6) | | | |
| Comparative Example 2 | Codeine | IPA (22) | - | γ-MnO₂ | 4.5 | NA | ∼34 |
| | phosphate (1) | H₂O (22) | | (3.2) | | | |
| Example 3 | Codeine | IPA (14) | 6N HCl | γ-MnO₂ | 1.6 | 80 | 91.8 |
| | phosphate (0.5) | H₂O(14) | (0.5) | (1.6) | | | |
| Example 4 | Codeine | IPA(40) | 6N HCl | γ-MnO₂ | 1.44 | NA | 60.3 |
| | (2) | H₂O (40) | (2) | (4) | | | |
| Example 5 | Codeine | IPA (40) | 6N HCl | γ-MnO₂ | 1.3 | 86 | ∼91.8 |
| | (2) | H₂O (40) | (3) | (7) | | | |
| Example 6 | Codeine | IPA(10) | 6N HCl | γ-MnO₂ | 0.73 | 75 | 82 |
| | (0.5) | H₂O(10) | (2) | (15) | | | |
| Example 7 | Codeine | IPA(10) | 6N HCl | γ-MnO₂ | 1.16 | 92 | ∼97 |
| | (0.5) | H₂O(10) | (1) | (1.5) | | | |
| Example 8 | Codeine | IPA (10) | 6N HCl | γ-MnO₂ | 1.41 | 95 | ∼95 |
| | (0.5) | H₂O(10) | (0.75) | (1.5) | | | |
| Example 9 | Codeine | CH₃CN (10) | 6N HCl | γ-MnO₂ | 0.9 | 89.7 | ∼93 |
| | (0.5) | H₂O(10) | (0.75) | (1.5) | | | |
| Comparative Example 10 | Codeine | IPA(10) | 6N HCl | γ-MnO₂ | <0.1 | 50.4 | ∼71 |
| | (0.5) | H₂O(10) | (4) | (1.5) | | | |
| Example 11 | Codeine | Acetone (10) | 6N HCl | γ-MnO₂ | 0.92 | 95.2 | 94 |
| | (0.5) | H₂O(10) | (0.75) | (1.5) | | | |
| Example 12 | Codeine | THF (10) | 6N HCl | γ-MnO₂ | 1.21 | 89 | 93 |
| | (0.5) | H₂O(10) | (0.75) | (15) | | | |
| Comparative Example 13 | Codeine | NMP | - | γ-MnO₂ | - | NA | ∼21.6 |
| | (0.5) | (20) | | | | | |
| Example 14 | Codeine | NMP (10) | 6N HCl | γ-MnO₂ | 1.45 | NA | ∼90 |
| | (0.5) | H₂O(10) | (1) | | | | |
| Example 15 | Codeine | Acetone (200) | 6N HCl | γ-MnO₂ | 1.02 | 83.5 | 95.5 |
| | (11.45) | H₂O(200) | (16) | (36) | | | |
| Example 16 | Codeine | Acetone (10) | 6N HCl | Activated | 0.87 | NA | ∼45 |
| | (0.5) | H₂O(10) | (0.75) | 85% MnO₂ (Aldrich) | | | |
| Comparative Example 17 | Codeine | IPA (25) | - | γ-MnO₂(3) | - | NA | ∼41 |
| | (1) | CH₂Cl₂ (25) | | | | | |
| NA = Not Available (the product was not isolated) | | | | | | | |

### Preparation of γ-Manganese Dioxide

Manganese (II) sulphate monohydrate (140g) was dissolved in 2.66 litres of water and heated to 60°C. Potassium permanganate (97.3g) in 1.85 litre of water was added over a period of 15 minutes and stirred at 60°C for 1 hour, until manganese dioxide precipitated out. The reaction mixture was filtered and the residue was washed with deionised water until no sulphate ion was present. The solid was dried under suction for 2 hours followed by drying at 70°C under vacuum to a constant weight (∼8 days) to give 115 g of a dark brown powder.

## Claims

1. A process for the preparation of a compound of formula (I) 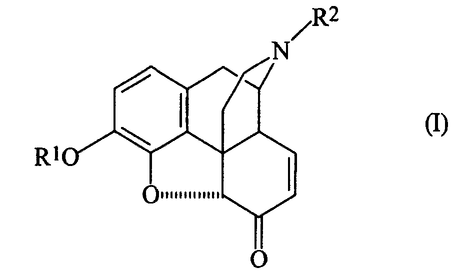 wherein R¹ is C₁₋₆ alkyl or a group 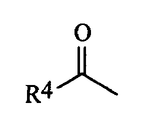 wherein R⁴ is C₁₋₆ alkyl, C₁₋₆ alkyl substituted by halogen or phenyl, or phenyl; and
R² is C₁₋₆ alkyl, allyl or C₁₋₆ alkyl substituted by cycloalkyl;
comprising the oxidation of a compound of formula (II), 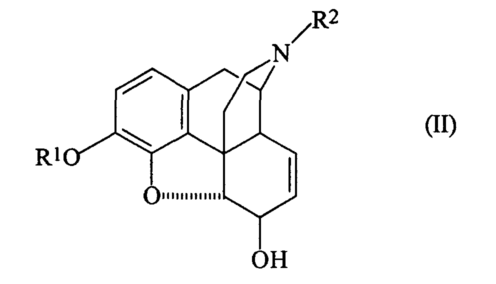 or a salt thereof, wherein R¹ and R² are as hereinbefore defined, and wherein the oxidation reagent is manganese dioxide, **characterised in that** the oxidation is carried out in an acidic environment, at a pH of greater than 0.1 and less than 4.5.

2. A process according to claim 1 wherein each of R¹ and R² may be the same or different and each is C₁₋₆ alkyl.

3. A process according to claim 2 wherein each of R¹ and R² may be the same or different and is methyl or ethyl.

4. A process according to claim 3 wherein R¹ and R² are both ethyl.

5. A process according to any one of claims 1 to 4 wherein when the compound of formula (II) is present as a salt, the salt is the phosphate salt.

6. A process according to any one of claims 1 to 5 wherein the oxidation is carried out at a pH of from 0.8 to 1.2.

## Patentansprüche

1. Verfahren zur Herstellung einer Verbindung der Formel (I) 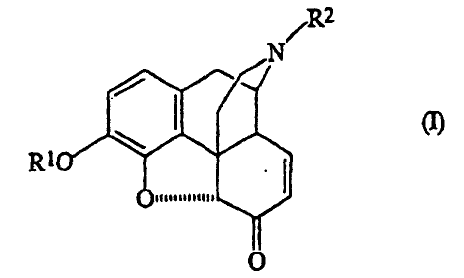 worin R¹ für C₁-C₆Alkyl oder die folgende Gruppe steht 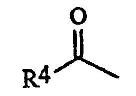 worin R⁴ für C₁-C₆Alkyl, durch Halogen oder Phenyl substituiertes C₁-C₆Alkyl oder fiir Phenyl steht und
R² für C₁-C₆Alkyl, Allyl oder durch Cycloalkyl substituiertes C₁-C₆Alkyl steht,
durch Oxidation einer Verbindung der Formel (II) 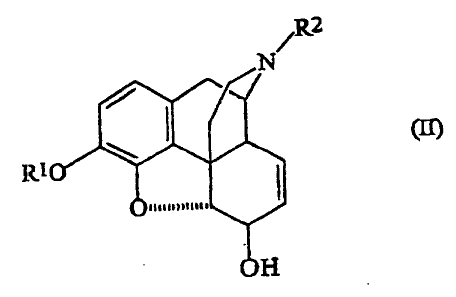 oder eines Salzes hiervon, worin R¹ und R² wie oben definiert sind, wobei das Oxidationsmittel Mangandioxid ist,
**dadurch gekennzeichnet, dass** die Oxidation in einer sauren Umgebung bei einem pH-Wert von über 0,1 und weniger als 4,5 durchgeführt wird.

2. Verfahren nach Anspruch 1, worin R¹ und R² gleich oder verschieden sein können und jeweils für C₁-C₆Alkyl stehen.

3. Verfahren nach Anspruch 2, worin R¹ und R² gleich oder verschieden sein können und für Methyl oder Ethyl stehen.

4. Verfahren nach Anspruch 3, worin R¹ und R² beide für Ethyl stehen.

5. Verfahren nach einem der Ansprüche 1 bis 4, worin, falls die Verbindung der Formel (II) als ein Salz vorliegt, dieses Salz das Phosphatsalz ist.

6. Verfahren nach einem der Ansprüche 1 bis 5, worin die Oxidation bei einem pH-Wert von 0,8 bis 1,2 durchgeführt wird.

## Revendications

1. Procédé de préparation d'un composé de formule (I) 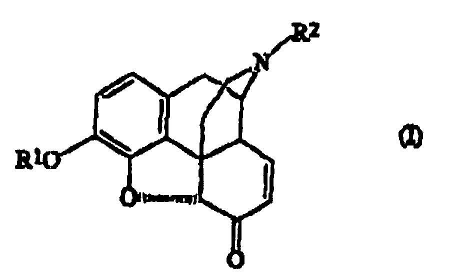 dans laquelle R¹ est un alkyle en C₁₋₆ ou un groupe 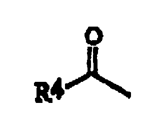 dans lequel R⁴ est un alkyle en C₁₋₆, un alkyle en C₁₋₆ substitué par un halogène ou un phényle, ou un phényle, et
R² est un alkyle en C₁₋₆, un allyle ou un alkyle en C₁₋₆ substitué par un cycloalkyle ;
comprenant l'oxydation d'un composé de formule (II) 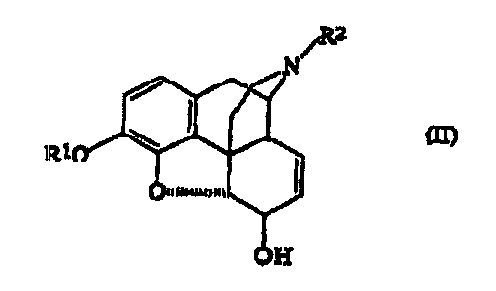 ou d'un sel de celui-ci, dans lequel R¹ et R² sont comme défini ci-dessus, et dans lequel le réactif d'oxydation est du dioxyde de manganèse, **caractérisé en ce que** l'oxydation est réalisée dans un environnement acide, à un pH supérieur à 0,1 et inférieur à 4,5.

2. Procédé selon la revendication 1, dans lequel chacun des R¹ et R² peut être le même ou différent et chacun est un alkyle en C₁₋₆.

3. Procédé selon la revendication 2, dans lequel chacun des R¹ et R² peut être le même ou différent et est un méthyle ou un éthyle.

4. Procédé selon la revendication 3 dans lequel R¹ et R² sont tous deux de l'éthyle.

5. Procédé selon l'une quelconque des revendications 1 à 4, dans lequel, lorsque le composé de formule (II) est présent sous forme de sel, le sel est un sel de phosphate.

6. Procédé selon l'une quelconque des revendications 1 à 5, dans lequel l'oxydation est réalisée à un pH de 0,8 à 1,2.
